(19) Europäisches Patentamt
European Patent Office
Office européen des brevets

(11) Publication number: 0 049 060
A1

# (12) EUROPEAN PATENT APPLICATION

(21) Application number: 81304085.4

(22) Date of filing: 08.09.81

(51) Int. Cl.³: C 07 D 233/60, A 61 K 31/415

(30) Priority: 13.09.80 GB 8029682
22.01.81 GB 8101924
24.03.81 GB 8109167

(43) Date of publication of application: 07.04.82
Bulletin 82/14

(84) Designated Contracting States: BE CH DE FR IT LI NL SE

(71) Applicant: BEECHAM GROUP LIMITED, Beecham House Great West Road, Brentford, Middlesex (GB)

(72) Inventor: Baggaley, Keith Howard, 3 Blackstone Hill, Redhill Surrey (GB)
Inventor: Watts, Eric Alfred, 217 Waterhouse Moor, Harlow Essex (GB)

(74) Representative: Stott, Michael John et al, European Patent Attorney Beecham Pharmaceuticals Yew Tree Bottom Road, Epsom Surrey KT18 5XQ (GB)

## (54) Imidazoles.

(57) A compound of the formula (I):

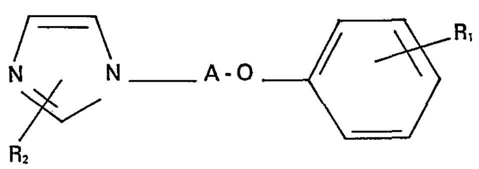

(I)

or a pharmaceutically acceptable salt thereof, wherein:

A is an alkylene group of 2 to 6 chain carbon atoms, one or two of which chain carbon atoms may be branched by one or two $C_{1-4}$ alkyl groups;

$R_1$ represents hydrogen, or one or more halogen, $C_{1-4}$ alkyl, $C_{1-4}$ alkoxy or $CF_3$; and

$R_2$ represents $C_{1-4}$ alkyl; has useful antidepressant activity.

EP 0 049 060 A1

IMIDAZOLES

This invention relates to novel imidazoles having anti-depressant activity, to pharmaceutical compositions containing these imidazoles, and to a process for the preparation of these imidazoles.

In J.C.S. Perkin I, p 1670, 1975, is described the preparation of a group of imidazoles of formula (A):

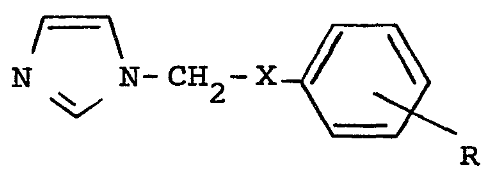

(A)

wherein X is O or S and R has a range of values. There is no suggestion in this paper that the compounds of formula (A) might have pharmaceutical utility.

The compounds of formula (A) wherein R is hydrogen and X is either $-CH_2-O-$ or $-CH_2-CH_2-O-$ are known compounds as shown in U.S. Patent No. 3,534,061 and Bull. Soc. Chim. Fr. 1976 (11-12, Pt. 2), 1861-4. There is again no suggestion in these publications that these compounds might have pharmaceutical activity.

UK Patent Application No 2018136A discloses that compounds of the formula (B):

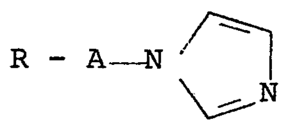

(B)

wherein

(i) A is a straight or branched alkylene group having 1, 2, 3 or 4 carbon atoms, and R is a naphthyl, tetrahydronaphthyl heterocyclyl, arylthio, arylalkylthio, aryloxy, arylalkyloxy, arylhydroxymethylene, arylcarbonyl, arylalkylcarbonyl, alkyloxy, alkylthio group or a cycloalkyl or cycloalkenyl group of from 4 to 9 carbon atoms substituted by a group other than a hydrocarbon group;

(ii) A is a $-SO_2-$ group, and R is an aryl or a heterocyclyl group;

(iii) A is a chemical bond and R is a heterocyclyl or substituted phenyl group, or

(iv) A is a straight or branched alkylene group having 1, 2 or 3 carbon atoms and R is halo-substituted phenyl group; or a pharmaceutically acceptable acid addition salt of such an imidazole are useful in the treatment or prophylaxis of thromboembolic conditions.

UK Patent Application No 2038821A discloses that compounds of the formula (C):

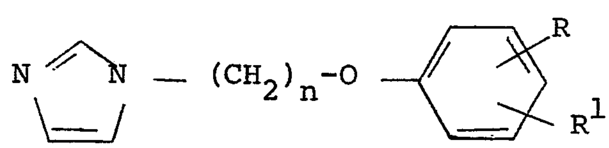

(C)

wherein:

R is $NO_2$, CN, $SO_2NH_2$, $C_1$-$C_4$ alkanoyl, $CO_2R^2$, $CH_2CO_2R^2$, $OCH_2CO_2R^2$, $CONHR^3$, $CH_2CONHR^3$, $OCH_2CONHR^3$, $CON(R^4)_2$, $CH_2CON(R^4)_2$, $OCH_2CON(R^4)_2$, $NHR^5$, $CH_2NHR^5$, tetrazolyl, $CH_2$-tetrazolyl and $OCH_2$-tetrazolyl;

$R^1$ is H, $C_1$-$C_4$ alkyl, $C_1$-$C_4$ alkoxy or halogen;

$R^2$ is H or $C_1$-$C_4$ alkyl;

$R^3$ is H, $C_1$-$C_4$ alkyl, $C_1$-$C_4$ alkanoyl, $C_1$-$C_4$ alkylsulphonyl, CN, benzoyl or benzenesulphonyl; the phenyl ring in said benzoyl or benzenesulphonyl groups being optionally substituted with one or more $C_1$-$C_4$ alkyl, $C_1$-$C_4$ alkoxy or $CF_3$ groups or halogen atoms; each $R^4$ is $C_1$-$C_4$ alkyl or two groups $R^4$ together with the nitrogen atom to which they are attached form a pyrrolidino, piperidino or morpholino group;

$R^5$ is H, $C_1$-$C_4$ alkanoyl, $C_1$-$C_4$ alkoxycarbonyl, carbamoyl, $C_1$-$C_4$ alkylcarbamoyl;

and n is 2 or 3;

and the pharmaceutically acceptable acid addition salts thereof and bioprecursors therefor; are able to selectively inhibit the action of the thromboxane synthetase enzyme without significantly inhibiting the action of the prostacyclin synthetase or cyclooxygenase enzymes.

West German Patent Application No 2836330 discloses a compound of formula (D):

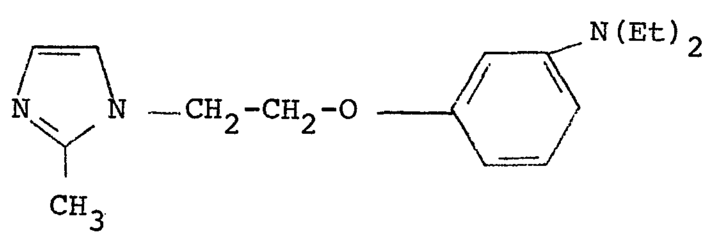

(D)

for use as a dye intermediate.

A class of alkyl imidazoles has now been discovered having useful mood-modifying for example anti-depressant activity.

Accordingly the present invention provides a compound of the formula (I):

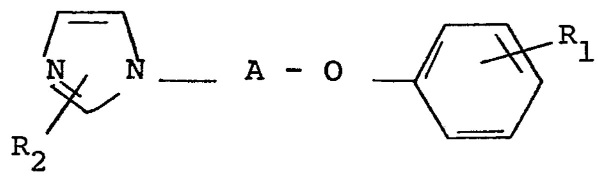

(I)

or a pharmaceutically acceptable salt thereof, wherein:

A is an alkylene group of 2 to 6 chain carbon atoms, one or two of which chain carbon atoms may be branched by one or two $C_{1-4}$ alkyl groups;

$R_1$ represents hydrogen, or one or more halogen, $C_{1-4}$ alkyl, $C_{1-4}$ alkoxy or $CF_3$; and

$R_2$ represents $C_{1-4}$ alkyl.

The compounds of the formula (I) suitably are in the form of their acid addition salts.

Conventional organic or inorganic acids may be used to form the addition salts, for example, phosphoric, hydrochloric, hydrobromic, acetic, lactic, citric, tartaric, propionic, methanesulphonic and maleate.

A may suitably be $-(CH_2)_2-$, $-(CH_2)_3-$ or $-(CH_2)_4-$, more suitably $-(CH_2)_2-$ or $-(CH_2)_3-$, preferably $-(CH_2)_2-$. When A is branched then normally only one carbon in the chain will be branched, and the branching will be by one or two methyl groups. Most suitably A is straight chain.

Suitable examples of optional phenyl substituents $R_1$ include chloro, bromo and iodo; methyl and ethyl; methoxy and ethoxy; and $CF_3$.

Often the phenyl moiety, when substituted, will be mono- or di-substituted. Particularly suitable phenyl substituents include alkyl such as methyl, and halogen such as chloro. Most usefully $R_1$ will represent di-halo subsitution, such as di-chloro substitution. Particularly suitably $R_1$ will represent two substituents, at the 2- and 4-positions respectively.

Suitable examples of $R_2$ include methyl and ethyl, preferably methyl. Preferably $R_2$ will be in the 2-position.

From the aforesaid it will be appreciated that one particularly useful sub-group of compounds within formula (I) are of formula (II):

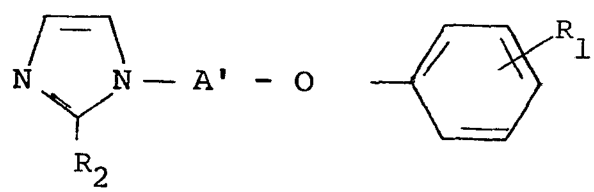

(II)

wherein A' is $-(CH_2)_n-$ wherein n is 2 or 3, which alkylene chain may be branched by one or two methyl groups.

Suitable and preferred examples of the variables in formula (II) are as described above in relation to the corresponding variables in formula (I).

By way of example, suitably the phenyl is unsubstituted, or mono- or di-substituted by halogen or alkyl.

Within formula (II) there is a particularly preferred sub-group of compounds of formula (II)':

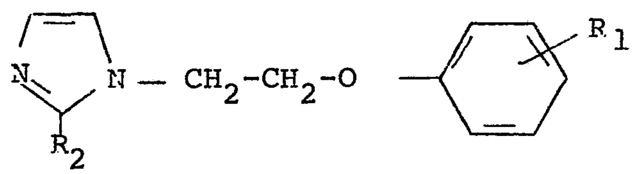

(II)'

and pharmaceutically acceptable salts thereof.

In formula (II)' suitable and preferred values for $R_1$ and $R_2$ are as described in relation to formula (I).

Particularly suitably in formula (II)' the phenyl is disubstituted by halogen; or mono substituted by halogen and mono substituted by alkyl. Preferably in formula (II)' the phenyl is 2- and 4-disubstituted by halogen, such as chloro and/or bromo.

A preferred compound of this invention has the formula (II)":

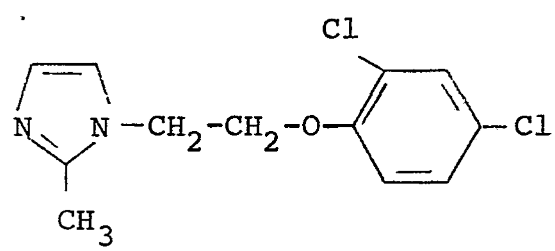

(II)"

The compounds of this invention may be used in the treatment of mental disorders, for example depression. They will normally be administered in the form of pharmaceutical compositions. Accordingly the invention also provides a pharmaceutical composition which composition comprises a compound of the formula (I), or a pharmaceutically acceptable salt thereof, and a pharmaceutically acceptable carrier.

The pharmaceutical compositions of the invention may be in a form suitable for oral or for parenteral use. Generally, injectable forms are required for emergency use while oral forms are preferred for prophylaxis or maintenance therapy.

Injectable forms may be in the form of solids for dissolution in sterile water or acceptable oils. Preferably this injectable composition comprises the medicament in salt form optionally together with conventional formulations aids such as acceptable preservatives, buffers and the like.

Suitable oral forms include tablets and capsules. Tablets of this invention include the medicament together with conventional additives such as lubricants, disintegrants, binder, fillers and the like. Capsules may be of any conventional material, for example, hard gelatin and may contain conventional additives as well as the medicament.

Normally the medicament will be used at a dose of 0.1mg/kg/day to 20 mg/kg/day as single or multiple doses. Normally individual dosage units will contain from 1 mg to 1,000 mg, preferably 2 mg to 750 mg.

The invention also provides a method of treatment of depression, which method comprises administering to the sufferer an effective amount of a compound of this invention.

The invention also provides a process for the preparation of the compounds of the formula (I), which process comprises reacting a compound of formula (III):

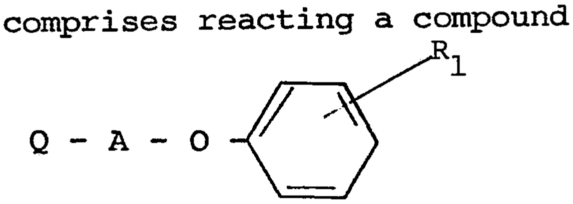

(III)

wherein Q is a group readily displaceable by a nucleophilic centre, and A is as defined in formula (I), with a compound of formula (IV) or a salt thereof:

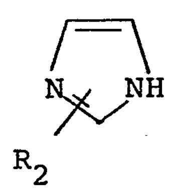

(IV)

Suitable groups Q include conventional good leaving groups such as Cl, Br, I, $O.SO_2CH_3$, $O.SO_2Ph$ and $O.SO_2C_6H_4CH_3$.

Preferred groups Q include Br and $O.SO_2C_6H_4CH_3$.

Compounds of the formula (I) are preferably prepared by the reaction of a phenoxyalkyl bromide of the formula (V):

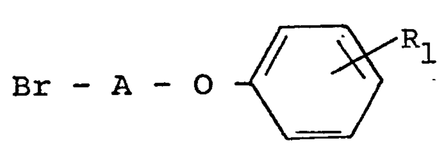

(V)

and the sodium salt of an imidazole of the formula (IV).

Such reactions are generally carried out in inert organic solvents such as n-butanol, dimethylformamide, dimethylsulphoxide and the like at a non-extreme temperature. Especially suitable temperatures for the reaction include slightly elevated temperatures, for example, from 20 to 140°C and preferably from about 50 to 120°C.

The invention also provides a second process for the preparation of the compounds of formula (I), which process comprises reacting a compound of formula (VI):

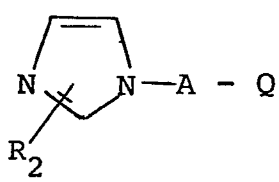

(VI)

with a salt of a compound of the formula (VII):

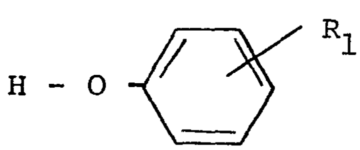

(VII)

The variable groups in the compounds of formulae (VI) and (VII) are as hereinbefore defined.

This second reaction of the invention is suitably carried out under conditions described above for the first reaction.

The following Examples illustrate the invention.

Example 1

1-(4'-Chlorophenoxyethyl)-2-methyl imidazole

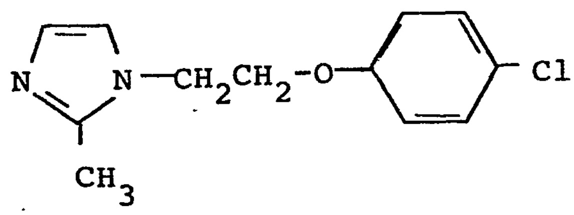

p-Chlorophenol (32.0 g, 0.25 mole) was dissolved with stirring in water (30 ml) containing sodium hydroxide (10 g, 0.25 mole) and added dropwise to excess refluxing ethylene dibromide (94 g, 0.5 mole). The reaction mixture was heated under reflux for two hours, cooled and extracted into ether (3 x 150 ml). The combined organic extracts were washed with water, dried over $Na_2SO_4$, filtered and evaporated to dryness to yield an oil. Fractionation under reduced pressure yielded p-chlorophenoxyethyl bromide (26.24 g, 46%) as a colourless oil $bp_{0.2}$, 98-106°C.

2-Methylimidazole (4.1 g, 0.05 mole) was dissolved in dimethylformamide (40 ml). Sodium hydride (2.3 g, of 60% w/w dispersion in oil) (equivalent to 1.4 g, 0.058 mole) was added portionwise with stirring. 4-Chlorophenoxyethylbromide (13.5 g, 0.058 mole) in dimethylformamide (25 ml) was added dropwise and the reaction mixture was heated on a water bath (100°C) for 3 hours. The reaction mixture was cooled and evaporated in vacuo to a beige oil. This oil was dissolved in ether and filtered to remove precipitated sodium bromide. The etheral filtrate was evaporated in vacuo to give a brown oil (8.00 g).

A solution in ether was treated with ethereal hydrogen chloride to give the hydrochloride (5 g) as a white solid, mp 152-154$^{o}$C.

Additional Characterising Data

Found: C, 52.68; H, 5.00; N, 9.93; Cl, 25.95. $C_{12}H_{14}N_2OCl_2$ requires: C, 52.74; H, 5.12; N, 10.26 and Cl, 26.00%.

Example 2

1-(4'-Chlorophenoxypropyl)-2-methyl imidazole

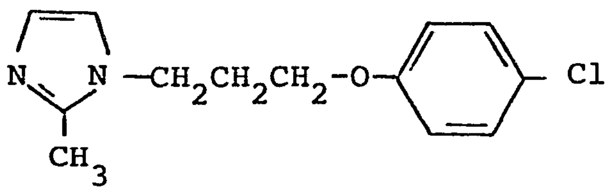

4-Chlorophenol (32 g), 1,3-dibromopropane (101 g) and sodium hydroxide (10 g) were heated in water (30 ml) in a manner described in Example 1 to give 4-chlorophenoxypropyl bromide (37 g) as an oil, $bp_{0.2\ mm}$ 128-134°C.

2-Methylimidazole (4.1g, 0.05 mol), 4-chlorophenoxy propyl bromide (14.5 g, 0.058 mole) and sodium hydride (2.3g of 60% dispersion in oil equivalent to 1.4 g, 0.058 mol) in dimethylformamide (70 ml) were reacted in a manner described in Example 1 to give 1-(4'-chlorophenoxypropyl)-2-methyl imidazole (10 g) purified by column chromatography on alumina.

A solution in ether was treated with ethereal hydrogen chloride to give the hydrochloride (7.9 g), mp 166-168°C.

Additional Characterising Data

Found: C, 54.36; H, 5.85; N, 9.49; Cl, 24.76. $C_{13}H_{16}N_2OCl$ requires: C, 54.36; H, 5.57; N, 9.76 and Cl, 24.74%.

Example 3

1-(4'-Chlorophenoxybutyl)-2-methyl imidazole

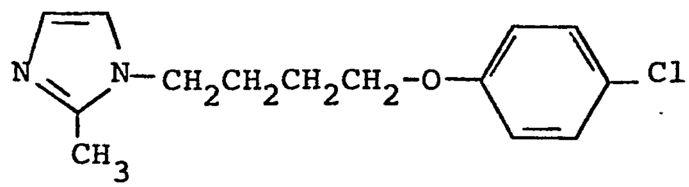

4-Chlorophenol (16 g), 1,4-dibromobutane (53.9 g) and sodium hydroxide (5 g) were heated in water (15 ml) in a manner described in Example 1 to give 4-chlorophenoxybutyl bromide (16 g).

2-Methylimidazole (4.1 g, 0.05 mol), 4-chloro phenoxybutyl bromide (16 g, 0.058 mol) and sodium hydride (2.3 g of 60% w/w dispersion in oil equivalent to 1.4 g, 0.058 mol) were reacted in dimethylformamide (75 ml) in a manner described in Example 1 to give 1-(4'-chlorophenoxybutyl)-2-methyl imidazole as a brown oil crystallising to a yellow solid.

A solution in ether was treated with ethereal hydrogen chloride to give the hydrochloride (6.7 g) as a white solid, mp 138-140°C.

Additional Characterising Data

Found: C, 55.74; H, 5.98; N, 9.01; Cl, 23.52. $C_{14}H_{18}N_2OCl_2$ requires: C, 55.81; H, 5.98; N, 9.30 and Cl, 23.58%.

Examples 4 to 9

The following compounds were prepared by analogous methods to those already described in previous Examples:

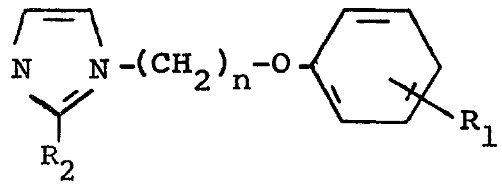

| Example | $R_1$ | n | $R_2$ | | mp$^{o}$C | Res Prev (lowest active dose) mg/kg p.o. | 5 HTP pot $ED_{100}$ or % change of control at x mg/kg po |
|---|---|---|---|---|---|---|---|
| 1 | (4-Cl | 2 | 2-Me | HCl salt | 152-154) | 10 | 5 |
| 2 | (4-Cl | 3 | 2-Me | " | 166-168) | 3 | 3 |
| 3 | (4-Cl | 4 | 2-Me | " | 138-140) | 100 | 16 |
| 4 | 4-F | 2 | 2-Me | " | 136-138 | 100 | 1 |
| 5 | 4-OMe | 2 | 2-Me | Free base | 54- 56 | 10 | - 52% @ 10 |
| 6 | 3-Cl | 2 | 2-Me | HCl salt | 192-194 | 100 | 7 |
| 7 | 2-Cl | 2 | 2-Me | " | 160-164 | 100 | + 12% @ 20 |
| 8 | 2-Cl,4-Cl | 2 | 2-Me | $HO_2C$=$CO_2H$ salt | 145.5-147.5 | 10 | 20 |
| 9 | 4-F | 3 | 2-Me | HCl salt | 150-153 | NT | 10 |

NT = Not tested

Example 8

1-(2',4'-Dichlorophenoxyethyl)-2-methylimidazole

For the sake of completeness, the full chemical preparation of this compound is noted below.

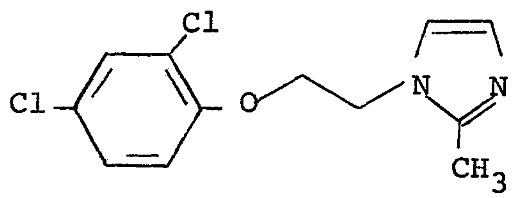

A solution of 2-methylimidazole (10.60 g, 0.13 mol) in dry DMF (60 ml) was added dropwise to a stirred suspension of sodium hydride (7.2 g, 0.15 mol, of 50% dispersion in oil) in dry DMF (20 ml) under an atmosphere of nitrogen. After 15 minutes, a solution of 2,4-dichlorophenoxyethyl bromide (40.5 g, 0.15 mol) in dry DMF (70 ml) was added dropwise, over 30 minutes, at room temperature. The resultant suspension was stirred for a further 3.5 hours at $100^{o}$ and then evaporated to dryness. The residue was washed with petrol (3 x 100 ml) and then partitioned between ether (1 000 ml) and water (800 ml). and the resultant solid removed by filtration. This residue was dissolved in dichloromethane, washed with water and dried ($Na_2SO_4$). Evaporation in vacuo gave 1-(2',4'-dichlorophenoxyethyl)-2-methylimidazole as a colourless solid (24.77g, 61%).

τ ($CDCl_3$): 2.67- 3.47 (5H, m, imidazole + aromatic protons); 5.73 (4H, br, s, $OCH_2.CH_2N$); and 7.60 (3H, s, $CH_3$).

A portion (22.54 g) in boiling acetone (100 ml) was treated with maleic acid (9.65 g) in cold acetone (100 ml) and the resultant precipitate (28.83 g) removed by filtration. Recrystallisation from acetone gave 1-(2',4'-dichlorophenoxyethyl)-2-methylimidazole monohydrogen maleate (22.23 g, 69%).

mp 145.5-147.5$^{o}$

Found: C, 49.94; H, 4.20; N, 7.20; Cl, 18.53.

$C_{16}H_{16}N_2O_5Cl_2$ requires: C, 49.63; H, 4.17; N, 7.23 and Cl, 18.31%

Example 10

1-(3',4'-Dichlorophenoxyethyl)-2-methylimidazole

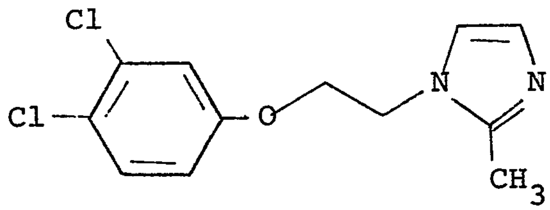

A solution of 3,4-dichlorophenol (2.7 g, 16.6 mmol) in water (50 ml) containing one equivalent of sodium hydroxide was stirred at 25$^{o}$ and ethylene oxide passed through the solution. After 30 minutes, the resultant suspension was extracted with ether (3 x 50 ml) and the combined organic layers washed with water (50 ml) and dried ($MgSO_4$). Evaporation in vacuo gave 2-(3',4'-dichlorophenoxy)ethan-1-ol as an oil (2.2g, 65%).

A solution of the foregoing alcohol (2.2 g, 10 mmol) in dry pyridine (50 ml) at 0$^{o}$ was treated with p-toluenesulphonyl chloride (4.04 g, 20 mmol) and the resultant solution placed in a refrigerator for 18 hours. The mixture was poured onto an ice/water mixture and stirred to give the tosylate as a white solid (3.30 g, 87%).

The foregoing tosylate (3.30 g, 9 mmol), 2-methylimidazole (0.69 g, 8.5 mmol) and sodium hydride (0.27 g of an 80% dispersion in oil equivalent to 9 mmol), in dry dimethylformamide (60 ml) were reacted in a manner similar to that described in Example 1 to give 1-(3',4'-dichlorophenoxyethyl)-2-methylimidazole as a solid (2.1 g, 88%). This was converted into the hydrochloride salt and recrystallised from ethanol-ether.

(Yield: 1.3 g, 48%), mp 208-210°.

Found: C, 47.12; H, 4.41 and N, 9.21. $C_{12}H_{13}N_2OCl_3$ requires: C, 46.82; H, 4.22 and N, 9.10%.

The following compounds were prepared according to the procedure described for Example 1 and purified as their monomaleate salts.

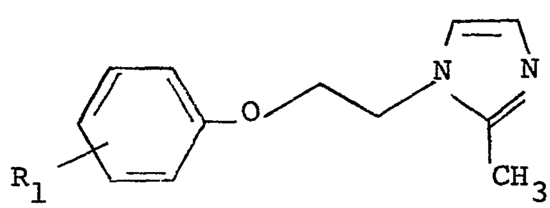

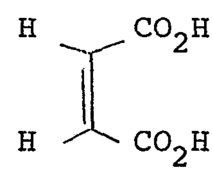

<u>Example 11</u> $R_1$ = 3,5-diCl

1-(3',5'Dichlorophenoxyethyl)-2-methylimidazole maleate.

mp 125-128$^{o}$ (from acetone-ether).
Found: C, 49.69; H, 4.06; N, 7.09; Cl, 18.30.
$C_{16}H_{16}N_2O_5Cl_2$ requires: C, 49.63; H, 4.17; N, 7.23 and Cl, 18.31%.

τ ($d^6$DMSO): 2.30-2.96 (5H, m, aromatic); 3.92 (2H, s, maleate); 5.34-5.70 (4H, m, 2 x $CH_2$); 7.34 (3H, s, $CH_3$).

<u>Example 12</u> $R_1$ = 2,3-diCl

1-(2',3'-Dichlorophenoxyethyl)-2-methylimidazole maleate

mp, 106-110° (from methanol-ether).
Found: C, 49.43; H, 3.98; N, 7.17; Cl, 18.16.
$C_{16}H_{16}N_2O_5Cl_2$ requires: C, 49.63; H, 4.17; N, 7.23; and Cl, 18.31%.

τ ($d^6$DMSO): 2.31-2.95 (5H, m, aromatic); 3.95 (2H, s, maleate); 5.30-5.70 (4H, 2 x 2H, t, 2 x $CH_2$); 7.32 (3H, s, $CH_3$).

<u>Example 13</u> $R_1$ = 2,5-diCl

1-(2',5'-Dichlorophenoxyethyl)-2-methylimidazole maleate.

mp, 97-100° (from acetone).
Found: C, 49.33; H, 4.04; N, 6.92; Cl, 17.54.
$C_{16}H_{16}N_2O_5Cl_2$ requires: C, 49.63; H, 4.17; N, 7.23 and Cl, 18.31%.

τ ($d^6$DMSO): 2.33-3.01 (5H, m, aromatic); 3.90 (2H, s, maleate); 5.25-5.65 (4H, 2x2H, t, 2x$CH_2$); 7.31 (3H, s, $CH_3$).

Example 14 $R_1$ = 2,6-diCl

1-(2',6'-Dichlorophenoxyethyl)-2-methylimidazole maleate.

mp, 86-87° (from acetone-ether).
Found: C, 49.14; H, 3.85; N, 7.10; Cl, 18.66.
$C_{16}H_{16}N_2O_5Cl_2$ requires: C, 49.63; H, 4.17; N, 7.23; and Cl, 18.31%.

τ ($d^6$-DMSO): 2.26-2.94 (5H, m, aromatic); 3.91 (2H, s, maleate); 5.30-5.80 (4H, m, 2 x $CH_2$); 7.35 (3H, s, $CH_3$).

Example 15 $R_1$ = 2,4 diBr

1-(2',4'-Dibromophenoxyethyl)-2-methylimidazole maleate.

mp, 147.5-149° (from acetone).
Found: C, 40.27; H, 3.15; N, 5.76; Br, 33.69.
$C_{16}H_{16}N_2O_5Br_2$ requires: C, 40.36; H, 3.39; N, 5.88 and Br, 33.57%.

τ ($d^6$DMSO): 2.16-2.60 (4H, m, C-3 + C-5 aromatic + imidazole ring); 2.93 (1H, d, J 9Hz, C-6 aromatic); 3.95 (2H, s, maleate); 5.30-5.75 (4H, m, 2 x $CH_2$); and 7.32 (3H, s, $CH_3$).

Example 16 $R_1$ = 2-Cl; 4-Br

1-(4'-Bromo-2'-chlorophenoxyethyl)-2-methylimidazole maleate.

mp, 148-149° (from acetone).
Found: C, 44.21; H, 3.63; N, 6.42; Cl, 8.03; Br, 18.10. $C_{16}H_{16}N_2O_5BrCl$ requires: C, 44.52; H, 3.74; N, 6.49, Cl, 8.21; Br, 18.51%.
τ ($d^6$DMSO): 2.26-2.60 (4H, m, C-3 + C-5 aromatic and imidazole ring); 2.87 (1H, d, J 9Hz, C-6 aromatic H); 3.89 (2H, s, maleate ); 5.30-5.75 (4H, m, 2 x $CH_2$) and 7.32 (3H, s, $CH_3$).

Example 17 $R_1$ = 3,4,5-tri-Cl

1-(3',4',5'-Trichlorophenoxyethyl)-2-methylimidazole maleate.

mp. 123-124° (from acetone)
Found: C, 45.40; H, 3.48; H, 6.56; Cl, 25.35. $C_{16}H_{15}N_2O_5Cl_3$ requires: C, 45.58; H, 3.59; N, 6.64; and Cl, 25.22%.

τ ($d^6$DMSO): 2.36 and 2.48 (2H, $AB_q$ J = 3Hz, imidazole H's); 2.66 (2H, s, C-2 + C-6 aromatic); 3.93 (3H, s, maleate); 5.35-5.70 (4H, m, 2x$CH_2$) and 7.35 (3H, s, $CH_3$).

Example 18 $R_1$ = 4-Cl, 2-Me

1-(4'-Chloro-2'-methylphenoxyethyl)-2-methylimidazole maleate.

mp. 111-112° (from acetone)
Found: C, 55.40; H, 5.10; N, 7.56, Cl, 9.68. $C_{17}H_{19}N_2O_5Cl$ required: C, 55.60; H, 5.22; N, 7.64; and Cl, 9.66%.

τ ($d^6$DMSO): 2.35 and 2.46 (2H, $AB_q$ J = 3Hz, imidazole H's); 2.69-2.82 (2H, m, C-3 + C-5 aromatic); 3.07 (1H, d, J 10Hz, C-6 aromatic); 3.95 (2H, s, maleate); 5.34;5.80 (4H, m, $2xCH_2$); 7.36 (3H, s, $CH_3$); and 7.94 (3H, s, $CH_3$-Ar).

Example 19 $R_1$ = 4-Cl; 3,5-di$CH_3$

1-(4'-Chloro-3',5'-dimethylphenoxyethyl)-2-methylimidazole maleate.

mp 90-93° (from acetone)
Found: C, 56.59; H, 5.64; N, 6.96, Cl, 9.30. $C_{18}H_{21}N_2O_5Cl$ required: C, 56.77; H, 5.56; N, 7.36; Cl, 9.31%.

τ: ($d^6$DMSO); 2.36 and 2.47 (2H, $AB_q$, imidazole); 3.20 (2H, s, C-2 + C-6 aromatic H's); 3.94 (2H, s, maleate ); 5.39-5.80 (4H, m, $2xCH_2$); 7.36 (3H, s, $CH_3$); and 7.72 (6H, s, 2 x $CH_3$-Ar).

Example 20 $R_1$=H

1-(2'-Phenoxyethyl)-2-methylimidazole.

mp. 77-78$^O$ (from ether-n-pentane)
Found: C, 71.12; H, 6.96; N, 13.82. $C_{12}H_{14}N_2O$ requires: C, 71.26; H, 6.98; and N, 13.85%.

τ ($CDCl_3$): 2.52-3.30 (7H, m, aromatic + imidazole H's); 5.82 (4H, s, 2x$CH_2$) and 7.58 (3H, s, $CH_3$).

Example 21 $R_1$=3-$CF_3$

1-(3'-Trifluoromethylphenoxyethyl)-2-methylimidazole maleate

mp. 86-90$^O$(from acetone)
Found: C, 52.46; H, 4.33; N, 7.14. $C_{17}H_{17}N_2O_5F_3$ requires: C, 52.85; H, 4.44; and N, 7.25%

τ ($d^6$DMSO) 2.26-2.50 (2H, $AB_q$, imidazole); 2.45-2.85 (4H, m, aromatic); 3.94 (2H, s, maleate); 5.30-5.65 (4H, m, 2x$CH_2$) and 7.33 (3H, s, $CH_3$).

The following compounds were prepared according to the procedure described for Example 1 and purified as their maleate salts.

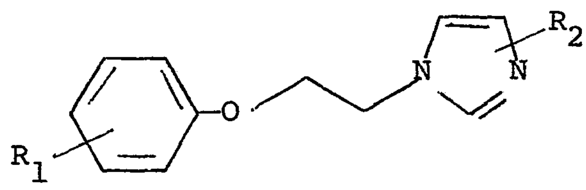

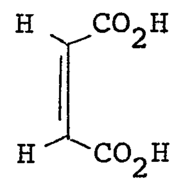

Example 22 $R_1$=2,4-diCl, $R_2$=4(5)-$CH_3$

1-(2',4'-Dichlorophenoxyethyl)-4(5)-methylimidazole maleate was prepared using 4(5)-methylimidazole and 2,4-dichlorophenoxyethyl bromide to give a 67:33 mixture of the two 4(5) methyl compounds.

mp 85-88$^{o}$ (from acetone-ether).
Found: C, 49.07; H, 3.89; N, 7.05; Cl, 18.30.
$C_{16}H_{16}N_2O_5Cl_2$ requires: C, 49.63; H, 4.17; N, 7.23 and Cl, 18.31%. $\tau$($d^6$DMSO): 1.13 (1H, t, J 1Hz;C-2 imidazole H); 2.34-2.88 (4H, m, aromatic); 3.92 (2H, s, maleate); 5.26-5.70 (4H, m, $2xCH_2$) and 7.61 and 7.73 (3H, 2d, 4 and 5 $CH_3$'s).

Example 23 $R_1$= 2,4-diCl, $R_2$= 2-$C_2H_5$

1-(2',4'-Dichlorophenoxyethyl)-2-ethylimidazole maleate was prepared using 2-ethylimidazole and 2,4-dichlorophenoxyethyl bromide.

mp 119-122$^{o}$ (from acetone.)
Found: C, 50.81; H, 4.32; N, 6.89; Cl, 18.07. $C_{17}H_{18}N_2O_5Cl_2$ requires: C, 50.89; H, 4.52; N, 6.98, Cl, 17.67%.

τ ($d^6$DMSO): 2.30-2.91 (5H, m, aromatic + imidazole protons); 3.89 (2H, s, maleate); 5.25-5.70 (4H, m, $2xCH_2$); 6.77-7.10 (2H, q, $\underline{CH_2}.CH_3$) and 8.68 (3H, t, $CH_2.\underline{CH_3}$).

Example 11, Process Variation

1-(3',5'-Dichlorophenoxyethyl)-2-methylimidazole

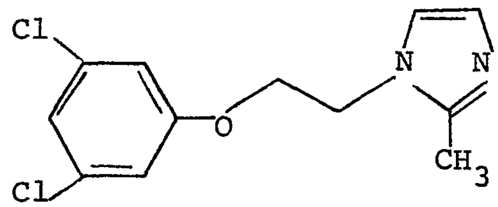

A mixture of 2-methylimidazole (2 g, 24 mmol) dibromoethane (15 ml) and glacial acetic acid (10 ml) was heated under reflux for 18 hours. The mixture was allowed to cool and then poured into 10% aqueous sodium hydroxide (150 ml). Extraction with ether (2x100 ml) and evaporation in vacuo gave 1-(2'-bromoethyl)-2-methylimidazole as a yellow gum (0.70 g). $\tau$ ($CDCl_3$); 5.70 and 6.57 (2 x 2H, t, $CH_2.CH_2Br$).

A solution of 3,5-dichlorophenol (650 mg, 3.68 mmol) in dry dimethylformamide (10 ml)was treated with 80% sodium hydride (120 mg, 4 mmol) under an atmosphere of nitrogen. After 15 minutes, a solution of the foregoing gum in 1:1 dry dimethylformamide-toluene (10 ml) was added dropwise and the mixture stirred at room temperature for 1 hour and then heated under reflux for a further 1 hour. The mixture was allowed to cool to room temperature, diluted with ethyl acetate (100 ml) and the solution was washed with 10% aqueous sodium hydroxide (3x30 ml), brine (30 ml) and dried ($Na_2SO_4$). Evaporation in vacuo gave a yellow oil (0.33 g) which was dissolved in ethyl acetate

(50 ml). The resultant solution was extracted with 5M-hydrochloric acid (50 ml). The pH of the aqueous layer was adjusted to 14 with 40% aqueous sodium hydroxide and extraction with ethyl acetate removed the product. Recrystallisation from ethyl acetate gave 1-(3',5'-dichlorophenoxyethyl)-2-methylimidazole (0.14 g, 15%). mp. 112-113$^{o}$.

Found: $M^{+}$ 270.0327. Calc for $C_{12}H_{12}N_2OCl_2$ 270.0325

$\tau$ ($CDCl_3$) : 2.92-3.43 (5H, m, aromatic + imidazole protons); 5.88 (4H, br s, 2x $CH_2$) and 7.60 (3H, s, $CH_3$).

Further Pharmacological Data

| Example | 5-HTP Potentiation, $ED_{100}$ or % Change of control at x mg/kg, p.o. |
| --- | --- |
| 10 | 12 |
| 11 | 18 |
| 12 | 28 |
| 13 | 19 |
| 14 | 9 |
| 15 | 34 |
| 16 | 96% at 30 |
| 17 | 70 |
| 18 | 10 |
| 19 | 77% at 100 |
| 20 | 10 |
| 21 | 26 |
| 22 | 138% at 30 |
| 23 | 53 |

## PHARMACOLOGICAL TEST METHODS

The ability of the compounds of this invention to potentiate catecholamines and 5-hydroxytryptamine was examined using the test methods (1) and (2) below, respectively. These tests are indicative of anti-depressant activity.

The results obtained in these tests are shown on pages 15 and 30.

### (1) Prevention of Reserpine-induced Hypothermia

The method of Spencer in "Anti-Depressant Drugs: Proceedings of the 1st International Symposium", p 184-204. Ed. Garattini, S and Dukes MNG, Excerpta Med Foundation, Amsterdam, 1967, was used. Groups of 5 mice were given oral doses of the test compound 24 hours, 18 hours and 2 hours before an intravenous injection of 1 mg/kg reserpine base. The oral temperatures of the mice were taken immediately before the administration of the reserpine and 6 hours afterwards. Mean temperatures of groups given test compounds are compared with the mean temperatures of the reserpinised controls using the Students t test. Compounds causing significant difference at the level $p<0.05$ are considered active at that dose.

### (2) Potentiation of the Effects of 5-Hydroxytryptophan

5 HTP Potentiation was measured in mice using the potentiation of the anti-convulsant activity of 5 HTP originally described by Buus Lassen J., (1975), Psychopharmacol, 42, 21-6. Mice (10/group) were pretreated with the compounds under investigation and 30' later received a sub-threshold level dose of L-5 HTP (100 mg/kg ip). 30 minutes later they were subjected to electroshock and the shock level (in V) required to cause tonic extensor spasm in 50% of the mice determined for all groups.

The dose of compounds required to increase the elevation of electroshock threshold due to 5HTP by 100% is determined graphically.

(3) Some of the compounds of the invention have been shown to be monoamine oxidase inhibitors.

(4) In other initial tests the compound of formula (II)":

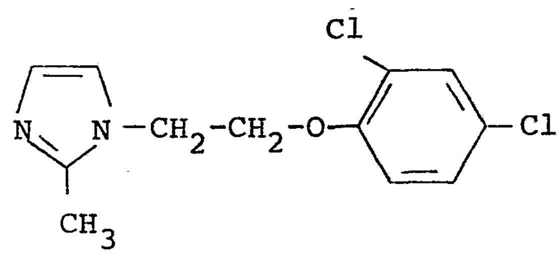

(II)"

has been shown to be a selective and reversible inhibitor of the A-form of monoamine oxidase. The compound also appears to have a low level of toxicity. There are also indications that the compound may have a usefully prolonged duration of action.

The $LD_{50}$ in mice of the compound is >900 mg/kg orally.

(5) In none of the tests reported herein were any toxic effects observed

CLAIMS

1. A compound of formula (I):

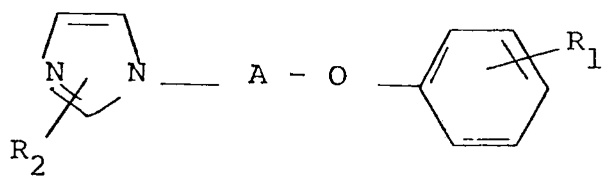

(I)

or a pharmaceutically acceptable salt thereof, wherein:

A is an alkylene group of 2 to 6 chain carbon atoms, one or two of which chain carbon atoms may be branched by one or two $C_{1-4}$ alkyl groups;

$R_1$ represents hydrogen, or one or more halogen, $C_{1-4}$ alkyl, $C_{1-4}$ alkoxy or $CF_3$; and

$R_2$ represents $C_{1-4}$ alkyl.

2. A compound according to claim 1, of formula (II):

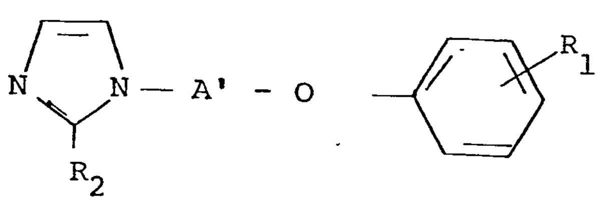

(II)

wherein A' is $-(CH_2)_n-$ wherein n is 2 or 3, which alkylene chain may be branched by one or two methyl groups
and the other variables are as defined in claim 1.

3. A compound according to claim 2, wherein the phenyl is di-substituted by halogen and/or alkyl.

4. A compound according to claim 3, wherein the phenyl substituents are 2- and 4-.

5. A compound according to claim 3 or 4, wherein A' is $-(CH_2)_2-$.

6. 1-(2',4'-Dichlorophenoxyethyl)-2-methylimidazole, or a pharmaceutically acceptable salt thereof.

7. A process for the preparation of a compound according to claim 1, which process comprises reacting a compound of formula (III):

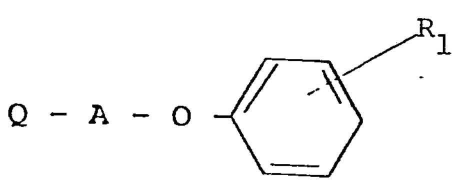

(III)

wherein Q is a group readily displaceable by a nucleophilic centre, and A and $R_9$ are as defined in claim 1, with a compound of formula (IV) or a salt thereof:

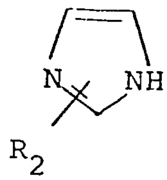

(IV)

wherein $R_2$ is as defined in claim 1,

or reacting a compound of formula (VI):

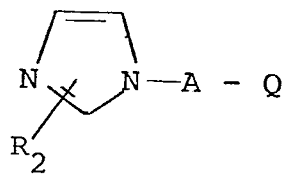

(VI)

wherein $R_2$, A and Q are as defined in formulae (III) and (IV),
with a salt of a compound of the formula (VII):

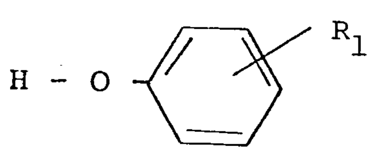

(VII)

wherein $R_1$ is as defined in formula (III).

8. A compound according to claim 1, for use in the treatment of depression.

European Patent Office

# EUROPEAN SEARCH REPORT

0049060

Application number

EP 81304085.4

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | GB - A - 1 554 841 (BAYER) * Claim 1 * ---- | 1 | C 07 D 233/60<br>A 61 K 31/415 |
| | | | TECHNICAL FIELDS SEARCHED (Int. Cl.³) |
| | | | C 07 D 233/00<br>A 61 K 31/00 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 18-12-1981 | BRUS |